Europäisches Patentamt

European Patent Office

Office européen des brevets

(19)

(11) Veröffentlichungsnummer: **0 173 241 B1**

(12) **EUROPÄISCHE PATENTSCHRIFT**

(45) Veröffentlichungstag der Patentschrift: 24.07.91

(21) Anmeldenummer: 85110484.4

(22) Anmeldetag: 21.08.85

(51) Int. Cl.⁵: **A61K 39/02**, A61K 39/116

(54) **Vakzine zur Behandlung der Harnwegsinfektionen.**

(30) Priorität: 31.08.84 CH 4181/84

(43) Veröffentlichungstag der Anmeldung:
05.03.86 Patentblatt 86/10

(45) Bekanntmachung des Hinweises auf die
Patenterteilung:
24.07.91 Patentblatt 91/30

(84) Benannte Vertragsstaaten:
**AT BE DE FR GB IT NL SE**

(56) Entgegenhaltungen:
EP-A- 0 048 881
US-A- 3 608 066

THE JOURNAL OF UROLOGY, Band 127, April
1982, Seiten 786-790, The Williams & Wilkins
Co., US; B. KAIJSER et al.: "Experimental
acute pyelonephritis caused by enterobacteria in animals. A review

(73) Patentinhaber: **Solco Basel AG**
**Gellertstrasse 18**
**CH-4052 Basel(CH)**

(72) Erfinder: **Stojkovic, Llubinko, Prof.**
**Oberwilerstrasse 64**
**CH-4054 Basel(CH)**
Erfinder: **Pavic, Rodmila, Dr.**
**Leimenstrasse 15**
**CH-4058 Basel(CH)**
Erfinder: **Spasojevic, Vera, Dr.**
**Hauptstrasse 24**
**CH-4302 Augst(CH)**

(74) Vertreter: **Frossard, Michel, Dr. et al**
**A. Braun, Braun, Héritier, Eschmann AG, Pa-**
**tentanwälte Holbeinstrasse 36-38**
**CH-4051 Basel(CH)**

EP 0 173 241 B1

## Beschreibung

Es sind bereits Vakzinen gegen verschiedene Krankheiten des Menschen oder der Tiere vorgeschlagen worden und es werden auch solche seit langem verwendet. Beispielsweise kann man gemäss CH-Patent 158'980 eine individuelle Mischvakzine für eine erkrankte Person herstellen, indem man die am jeweiligen Krankheitsherd vorhandenen Keime, z.B. den Tonsillen, der Vagina, dem Auswurf oder den Faeces entnimmt, wahllos züchtet und inaktiviert. Nach FR-Patent 2'034'743 kann ein Antigen verschiedenster Herkunft durch Versetzen eines entsprechenden flüssigen Extraktes mit Aluminiumtannat wasserunlöslich gemacht werden; das Produkt weist eine verzögerte Absorption und dadurch eine verlängerte Wirkung auf.

Eine Vakzine auf der Basis enteropathogener Stämme der Art Escherichia coli soll gemäss Europäischem Patent 28'172, US-Patent 4'338'298 oder Unlisted Drugs 30 (1978), 123 - Gletvax K88 zur passiven Immunisierung neugeborener Schlachttiere dienen. Die Vakzine wird dem Muttertier einige Zeit vor dem Wurf intramuskulär oder subkutan verabreicht und schützt die Kälber, Ferkel usw. nach der Geburt gegen den durch Colibakterien erzeugten Durchfall.

Auch für den Menschen sind Vakzinen auf der Basis von Colibakterien schon vorgeschlagen worden. Gemäss DE-Offenlegungsschrift 1'931'195 werden Mikroorganismen dem Auswurf von an Infektionen der Atemwege leidenden Personen entnommen, abgetötet und zu einer Vakzine in Form eines Aerosols aufgearbeitet; sie wird durch Inhalation verabreicht und soll zur Immuntherapie der Atemwege verwendet werden. Die Vakzine enthält neben vielen anderen Bakterienarten und -stämmen solche der Escherichia coli.

Eine weitere Vakzine besteht gemäss FR-Patent 2'397'839 aus abgetöteten oder abgeschwächten Keimen einer Anzahl von Bakterienarten mitsamt elf verschiedenen Stämmen der Escherichia coli; sie wird per os verabreicht und soll zur Immunisierung des Gastrointestinaltraktes dienen und vor allem die Gastroenteritis heilen bzw. dagegen schützen. Die Vakzine enthält ferner Methionin, Eisensalze, Vitamine, Lactobacillen usw.

Hingegen ist bisher keine Vakzine zustande gekommen, welche gegen die Infektionen der Harnwege spezifisch gerichtet und auf der Basis insbesondere der Escherichia coli zusammengesetzt wäre. Das Fehlen einer solchen Vakzine ist umso erstaunlicher, als diese Infektionen ein wichtiges Problem der Medizin darstellen - dies einerseits wegen deren häufiges Vorkommen, das trotz breiter Anwendung von Antibiotica und Chemotherapeutica nicht seltener wird, und andererseits wegen deren Neigung zu Rückfällen (Rückfall in über 80 % der Fälle) und zum chronischen Verlauf.

Der Grund des bisherigen Fehlens liegt offensichtlich in der riesigen Anzahl serologischer Typen der Escherichia coli. Diese Bakterien besitzen nämlich eine sehr komplexe Antigenstruktur, mit drei Antigen-Hauptgruppen: man kennt heute etwa 160 O-Antigentypen, über 60 K-Typen und über 60 H-Typen; ihre Kombination ergibt etwa 10'000 serologisch unterschiedliche Escherichia coli-Stämme. Bei einer derartigen Anzahl Stämme musste es von vornherein aussichtslos erscheinen, eine gegen Harnwegsinfektionen allgemein wirksame Vakzine aus einer einigermassen annehmbaren Anzahl Escherichia coli-Stämmen verwirklichen zu können.

Um den Anspruch auf allgemeine Wirksamkeit gegen Harnwegsinfektionen überhaupt zu verdienen, müsste eine derartige Vakzine in der Tat Immunität gegenüber der Cystitis, der Prostatitis, der Pyelitis und der Pyelonephritis gewährleisten, welcher bakteriellen Genese die Krankheit auch sei. Es ist aber klar, dass je breiter der Immunitätsbereich ist, umso grösser ist auch die Möglichkeit einer auf körpereigene Systeme übergreifenden Immunreaktion.

Bekanntlich tragen die Colibakterien Fimbrien (Pili, Fibrillae), welche für die Adhäsion der Colibakterien des Darms an der Darmschleimhaut verantwortlich sind; ohne die Mitwirkung der Fimbrien würden die Colibakterien vom Darm weggeschwemmt und dadurch das physiologische Gleichgewicht zwischen den Coli- und den anderen Bakterienarten im Darm schwer gestört werden. Von einer Vakzine auf der Basis von Colibakterien konnte man deshalb befürchten, dass die im Körper erzeugten Antikörper mit allen, gewöhnliche Fimbrien tragenden Systemen reagieren würden, somit auch mit den Colibakterien des Darms; die Folge wäre die erwähnte tiefgreifende Störung der Darmflora.

Ueberraschenderweise hat es sich nun dennoch möglich erwiesen, aus einer bescheidenen Anzahl Escherichia coli-Stämmen und einigen wenigen anderen Bakterienarten, die aus dem Harn von an einer Harnwegsinfektion leidenden Menschen isoliert worden sind, eine gegen solche Infektionen spezifisch und allgemein wirksame Vakzine herzustellen, welche jedoch entgegen den bisherigen Befürchtungen praktisch keine schädlichen Auswirkungen auf die Darmflora hat.

Erfindungsgemäss besteht die neue Vakzine aus einer Suspension in steriler isotonischer Lösung, welche (1) inaktivierte Bakterien, welche aus Kulturen von 8 bis 14 aus dem Harn von an einer Infektion der Harnwege leidenden Menschen isolierten uropathogenen Bakterienstämmen der Arten :

2

- Escherichia coli
- Klebsiella pneumoniae
- Proteus mirabilis
- Proteus morganii und
- Streptococcus faecalis

stammen und in einer Menge von etwa 50 Millionen bis 500 Millionen Keime je Stamm per ml vorliegen, wobei die Hälfte bis Dreiviertel der verwendeten Stämme der Art Escherichia coli angehören, und (2) Aluminiumphosphat in kolloidaler Form in einer Menge von 1,5 bis 10 mg AlPO$_4$ per ml enthält.

Die Herstellung der Vakzine erfolgt dadurch, dass man die oben erwähnten 8 bis 14 uropathogenen Bakterienstämme, welche aus dem Harn von an einer Infektion der Harnwege leidenden Menschen isoliert worden sind und von welchen die Hälfte bis Dreiviertel der Art Escherichia coli angehören, jeweils für sich allein auf einem geeigneten Nährboden züchtet, nach Abschluss der Züchtung das jeweils gebildete biologische Material abtrennt und nach bekannten Methoden inaktiviert, die aus den einzelnen Stämmen erhaltenen und inaktivierten Bakterien miteinander in solchen Mengen vermischt und mit einer sterilen isotonischen Lösung soweit verdünnt, dass etwa 50 Millionen bis 500 Millionen Keime je Stamm per ml vorliegen, und mit Aluminiumphosphat in kolloidaler Form bis zu einer Konzentration von 1,5 bis 10 mg AlPO$_4$ per ml versetzt.

Im folgenden wird die Erfindung ausführlich beschrieben.

Zunächst wurden von Personen, die an einer Harnwegsinfektion litten, Harnproben (Mittelstrahlurin) gewonnen und sofort auf ein MacConkey-Agar überimpft; nach der Beimpfung wurden die Agarplatten während 16 bis 18 Stunden bei 37 °C bebrütet; anschliessend wurden die gebildeten Kolonien isoliert und durch Bestimmung ihres biochemischen und biologischen Verhaltens identifiziert.

## Identifizierung von Streptococcus faecalis (Enterococcus)

Durch Gram-Färbung charackteristischer kleiner Kolonien und folgende Prüfungen :

| | |
|---|---|
| - Katalase (in Gegenwart von erhitztem Blut) | + |
| - Haemolyse | -/$\beta$ |
| - Wachstum bei 45 °C | + |
| - Wachstum bei pH 9,6 | + |
| - Wachstum in 6,5 % NaCl-Lösung | + |
| - Wachstum auf 40 % Galle | + |
| - Nachweis von Polysaccharid-Antigen D | + |

Folgende Kriterien wurden verwendet, um die Stämme von E.coli, Proteus und Klebsiella zu identifizieren :

3

| | E.coli | Proteus morganii | Proteus mirabilis | Klebsiella pneumoniae |
|---|---|---|---|---|
| Motilität | + | + | + | + |
| Wachstum in KCN-Medium | - | + | + | - |
| Citrat als C-Quelle | - | - | + | + |
| Kohlehydrat-Gas von Glucose | + | + | + | + |
| Säure von Lactose | + | - | - | + |
| von Sucrose | +(d) | - | + | + |
| von Maltose | + | - | - | + |
| von Mannit | + | - | - | + |
| von Trehalose | + | +(d) | + | + |
| von Xylose | +(d) | - | - | + |
| Gelatinehydrolyse | - | - | + | - |
| Indol | + | + | - | - |
| Urease | - | + | + | + |
| $H_2S$ von TSI (Dreifach-Zucker-Eisen-Agar) | - | - | + | - |
| Lysindehydrocarboxylase | + | - | - | + |

Die derart identifizierten Kolonien wurden auf Agarplatten während 24 Stunden bei 37 °C weiter wachsengelassen, dann in physiologischer Kochsalzlösung suspendiert, mittels Gram-Färbung auf Reinheit geprüft und trockengefroren. Die erhaltenen einzelnen Stämme sind wie folgt charakterisiert und mit den aus den Tabellen ersichtlichen Bezeichnungen versehen worden.

Fermentierung der Kohlehydrate

| Kohle-hydrat | Escherichia coli-Stamm | | | | | |
|---|---|---|---|---|---|---|
| | 455 UB | 525 UB | 560 UB | 616 UB | 654 UB | 719 UB |
| Lactose | + | + | + | + | + | + |
| Saccharose | 0 | 0 | 0 | + | + | + |
| Mannit | + | + | + | + | + | + |
| Maltose | + | + | + | + | + | + |
| Melibiose | + | + | + | + | + | + |
| Raffinose | 0 | 0 | 0 | 0 | 0 | 0 |
| Rhamnose | + | ± | + | + | + | + |
| Trehalose | + | + | + | + | + | + |
| Salicin | 0 | 0 | 0 | 0 | 0 | + |
| Ribose | + | + | + | + | + | + |
| Amygdalin | 0 | 0 | 0 | 0 | 0 | + |
| Galactose | + | + | + | + | + | + |
| Sorbit | + | + | + | + | + | + |
| Arabinose | + | + | + | + | + | + |
| Glucose | + | + | + | + | + | + |
| Mannose | + | + | + | + | + | + |
| Fructose | + | + | + | + | + | + |
| Adonit (Ribit) | 0 | 0 | 0 | 0 | 0 | 0 |
| Inosit | 0 | 0 | 0 | 0 | 0 | 0 |
| Dulcit | 0 | 0 | 0 | 0 | 0 | 0 |
| Cellobiose | 0 | 0 | 0 | 0 | 0 | + |
| Xylose | + | ± | + | + | + | + |

+ = Säure

Biochemische Eigenschaften

| Test | Escherichia coli-Stamm | | | | | |
|---|---|---|---|---|---|---|
| | 455 UB | 525 UB | 560 UB | 616 UB | 654 UB | 719 UB |
| Harnstoff | 0 | 0 | 0 | 0 | 0 | 0 |
| Hämolyse auf Blutagarplatten | 0 | 0 | 0 | 0 | 0 | + |
| Gelatine-hydrolyse | 0 | 0 | 0 | 0 | 0 | 0 |
| Citrat | 0 | 0 | 0 | 0 | 0 | 0 |
| Indol | + | + | + | + | + | + |
| $H_2S$ | 0 | 0 | 0 | 0 | 0 | 0 |
| Motilität | + | + | + | + | + | + |

+ = positiv in   24 Stunden

0 = negativ nach 72 Stunden

Fermentierung der Kohlehydrate

| Kohlehydrat | Klebsiella pneumoniae Kl 16 B | Proteus mirabilis 63 B | Proteus morganii 58 B |
|---|---|---|---|
| Lactose | 0 | 0 | 0 |
| Saccharose | + | 0 | 0 |
| Mannit | + | 0 | 0 |
| Maltose | + | 0 | 0 |
| Melibiose | 0 | 0 | 0 |
| Raffinose | 0 | 0 | 0 |
| Rhamnose | + | 0 | 0 |
| Trehalose | + | + | 0 |
| Salicin | + | 0 | 0 |
| Ribose | + | + | + |
| Amygdalin | 0 | 0 | 0 |
| Galactose | + | + | + |
| Sorbit | + | 0 | 0 |
| Arabinose | + | 0 | 0 |
| Glucose | + | + | + |
| Mannose | + | 0 | + |
| Fructose | + | 0 | + |
| Adonit (Ribit) | 0 | 0 | 0 |
| Inosit | 0 | 0 | 0 |
| Dulcit | 0 | 0 | 0 |
| Cellobiose | + | 0 | 0 |
| Xylose | + | + | 0 |

+ = Säure

Biochemische Eigenschaften

| Test | Klebsiella pneumoniae Kl 16 B | Proteus mirabilis 63 B | Proteus morganii 58 B |
|---|---|---|---|
| Harnstoff | 0 | + | + |
| Hämolyse auf Blutagarplatte | 0 | 0 | + |
| Gelatine-hydrolyse | 0 | + | 0 |
| Citrat | + | + | 0 |
| Indol | 0 | 0 | + |
| $H_2S$ | 0 | + | 0 |
| Motilität | 0 | + | + |

+ = positiv in 24 Stunden

0 = negativ nach 72 Stunden

Fermentierung der Kohlehydrate

| Kohlehydrat | Streptococcus faecalis 676 |
|---|---|
| Lactose | + |
| Saccharose | + |
| Mannit | + |
| Maltose | + |
| Melibiose | 0 |
| Raffinose | 0 |
| Rhamnose | + |
| Trehalose | + |
| Salicin | + |
| Ribose | + |
| Amygdalin | + |
| Galactose | + |
| Sorbit | + |
| Arabinose | 0 |
| Glucose | + |
| Mannose | + |
| Fructose | + |
| Adonit (Ribit) | 0 |
| Inosit | 0 |
| Dulcit | 0 |
| Cellobiose | + |
| Xylose | 0 |

+ = Säure

## Biochemische Eigenschaften

| Test | Streptococcus faecalis 676 |
|------|----------------------------|
| Harnstoff | 0 |
| Hämolysetyp | nicht hämolytisch |
| Gelatinehydrolyse | 0 |
| Reduktion der Litmus-milch | + |
| Indol | 0 |
| Aesculin | + |

## Wachstum auf Nährboden

| | |
|------|------|
| 40 % Galle | + |
| 6,5% Natriumchlorid | + |
| pH 9,6 | + |

## Temperaturresistenz

| | |
|------|------|
| 60 °C während 30 minuten | + |

+ = positiv

0 = negativ

Die morphologischen Eigenschaften der isolierten und oben beschriebenen Stämme lassen sich wie folgt zusammenfassen.

Escherichia coli:
Gramnegative Stäbchen, 1,1 - 1,5 x 2,0 - 6,0 $\mu$m (lebende Form), beweglich durch peritriche Flagellen.

Proteus mirabilis und P. morganii:
Gramnegative Stäbchen, 0,4 - 0,6 x 0,1 - 3,0 μm ohne Kapsel, beweglich mit peritriche Flagellen (nicht alle).

Klebsiella pneumoniae:
Gramnegative, nicht bewegliche Stäbchen 0,3 - 1,5 μm x 0,6 - 6,0 μm.

Streptococcus faecalis:
Gramnegative ovoide Kokken,0,5 - 1,0 μm, nicht beweglich.

Die Escherichia coli-Stämme wurden ferner auf ihre serologische Beschaffenheit untersucht und zu diesem Zweck zuerst auf dem Objektträger dem Agglutinationstest mit den polyvalenten OK-Seren A, B, C, D und E unterworfen. Die weitere serologische Typisierung (Bestimmung der O-, K- und H-Antigene) erfolgte nach den Vorschriften der Difco Laboratories, Detroit (MI, USA), 1976. Diese Identifizierung ergab folgendes Bild:

| Stamm | Bezeichnung | Serologischer Typ |
|---|---|---|
| Escherichia coli | Ec 455 UB | O 6 : K 13 : H 1 |
| Escherichia coli | Ec 525 UB | O 1 : K 1 : H 7 |
| Escherichia coli | Ec 560 UB | O 4 : K 3 : H 5 |
| Escherichia coli | Ec 616 UB | O 75 : ---- : H 5 |
| Escherichia coli | Ec 654 UB | R - Form |
| Escherichia coli | Ec 719 UB | Hämolytisch, nicht typisierbar |

Die oben genannten und definierten 10 Stämme sind am 8. August 1984 beim Centraalbureau voor Schimmelcultures Oosterstraat 1, 3740 AG Baarn (Niederlande) unter den Bezeichnungen CBS 516.84 bis CBS 525.84 deponiert worden und am 5. Dezember 1984 in die Deutsche Sammlung von Mikroorganismen, Grisebachstrasse 8, 3400 Göttingen (Bundesrepublik Deutschland) unter den Eingangsnummern DSM 3229 bis DSM 3238 übergeführt worden.

Zur Herstellung der Vakzine werden die Stämme auf einem festen oder flüssigen Nährboden einzeln gezüchtet; vorzugsweise wird ein fester Nährboden verwendet, beispielsweise Nähr-Agar (Nutrient Agar Difco), welcher folgende Zusammensetzung besitzt :

| | | |
|---|---|---|
| Fleischextrakt (Difco Beef Extract) | 3 | g/ℓ |
| Bacto-Pepton | 5 | g/ℓ |
| Natriumchlorid | 5 | g/ℓ |
| Bacto-Agar | . 15 | g/ℓ |

Das Medium wird 15 Minuten lang bei 121 °C sterilisiert; es weist einen pH-Wert von ca. 7,2 auf.

Für die Grossherstellung werden Roux-Flaschen, für die Herstellung im Laboratorium werden Petri-Schalen verwendet. Die Impfung wird mit Einsaatmaterial vorgenommen, von dem einige ml gleichmässig über die ganze Oberfläche verteilt werden. Die Roux-Flaschen werden mit einem Baumwollpropfen verschlossen und das Medium mit der beimpften Oberfläche nach unten gelagert. Die beimpften Kulturen werden 24 Stunden lang bei 37 °C bebrütet.

Der Bakterienrasen wird mit der notwendigen Menge (abhängig von der Wuchsdichte der Kultur im Gefäss) Phosphatpuffer-Kochsalzlösung (PBS) unter leichtem Schaukeln, ohne die Agaroberfläche zu verletzen, abgeschwemmt.

Von jeder Flasche oder jeder Petri-Schale wird ein Ausstrich gemacht, nach Gram gefärbt und auf Reinheit geprüft. Flaschen oder Petri-Schalen, die verunreinigt erscheinen, werden verworfen. Suspensionen die von einem Stamm und von einer Ernte stammen, werden über eine sterile Nylongaze zusammengeschüttet.

Die Inaktivierung wird, wie die Züchtung selber, für jeden Stamm einzeln vorgenommen. Sie kann durch Erhitzen bei einer Temperatur von etwa 55 bis 60 °C während etwa einer Stunde, durch Behandeln mit einer Formaldehydlösung oder durch Bestrahlung mit γ-Strahlen durchgeführt werden. Ihrer Einfachheit wegen wird die Inaktivierung durch Erhitzen bevorzugt.

Die durch die Züchtung erhaltenen Mengen werden vereinigt, im Wasserbad bei 60 °C während einer Stunde inaktiviert und nach der Hitze-Inaktivierung wird Phenol zugesetzt (Endkonzentration 0,35 %).

Eine Probe der Suspension wird für den Sterilitäts-Test und zur Prüfung auf Identität entnommen und die konzentrierte Vorratsmenge wird im Eisschrank aufbewahrt.

Wenn die Sterilitätstests nach 3 Tagen keine Verunreinigungen ergeben, wird der Herstellungsprozess weitergeführt. Der Sterilitätstest wird 14 Tage lang beobachtet, und wenn die Sterilitätsprobe ein Wachstum von irgendwelchen Organismen zeigt, wird diese Ernte eliminiert.

Die Inhalte der Vorratsbehälter (inaktivierte Suspensionen, die von einem Stamm herrühren) werden bei 3000 UpM 1 Stunde lang in einer gekühlten Zentrifuge (Sorvall 4 CR, Aufschwungrotor 2000) zentrifugiert, die überstehende Flüssigkeit wird entfernt und das Bakteriensediment in Kochsalzlösung, die 0,01 % Thiomersal enthält, suspendiert. Die Dichte der Vorratskonzentrate wird mit einem Trübungsstandard bestimmt, die Suspension wird beschriftet und bei 4 °c (± 2 °C) aufbewahrt.

Die konzentrierten Suspensionen der 10 Stämme von E. coli, Proteus mirabilis, Proteus morganii, Klebsiella pneumoniae und Streptococcus faecalis werden so gemischt, dass in 1 Milliliter enthalten sind :

E. coli, 6 Stämme

von jedem $2,5 \times 10^8$, insgesamt $1,5 \times 10^9$ Keime

Proteus mirabilis, 1 Stamm $\qquad$ $7,5 \times 10^7$ Keime

Proteus morganii, 1 Stamm $\qquad$ $7,5 \times 10^7$ Keime

Klebsiella pneumoniae, 1 Stamm $\quad 3 \quad \times 10^8$ Keime

Streptococcus faecalis, 1 Stamm $5 \quad \times 10^7$ Keime

Zusammen $\qquad\qquad$ $2 \quad \times 10^9$ Keime/ml

Die Endkonzentration wird durch Zusammenmischen der individuellen Vorratssuspensionen erhalten. Man erreicht die beschriebene Konzentration durch Verdünnung mit 0,15 M Phosphat-gepufferter Salzlösung, die 0,01 % Merthiolat (Thiomersal) enthält. Der Vorrat wird nun 24 Stunden lang bei Raumtemperatur gehalten und dann bei 4 °C (± 2 °C) aufbewahrt.

Da Aluminiumphosphat-Gel zugesetzt wird, muss die Konzentration entsprechend höher sein. Das Aluminiumphosphat-Gel wird folgendermassen hergestellt:

854 g Kalium-aluminiumsulfat • 12 $H_2O$ werden in 6 Liter Wasser gelöst und filtriert. Ferner werden 685 g Trinatriumphosphat • 12 $H_2O$ in 6 Liter Wasser aufgelöst. Die Aluminiumlösung wird bei einer Temperatur von 37 °C gehalten, da sie bei Raumtemperatur leicht übersättigt ist. Beide Lösungen werden gleichzeitig in 21 Liter Wasser hineingegossen. Der Niederschlag wird zentrifugiert, das Sediment in 13 Liter Wasser resuspendiert und die Suspension erneut zentrifugiert.

Das Sediment wird schliesslich resuspendiert, auf ein Gesamtvolumen von 8,1 Liter gebracht, mit 5 n-NaOH auf pH 6,0 eingestellt und während einer Stunde bei 121 °C autoklaviert. Diese Menge reicht aus, um 66 Liter einer Vakzine mit 3 mg $AlPO_4$/ml bzw. 0,66 mg Al/ml herzustellen.

Die adsorbierte Endvakzine wird nach Prüfung des pH-Wertes, der zwischen 6,2 und 7,0 liegen muss, in sterile pyrogenfreie Ampullen von 1 ml Fassungsvermögen in einer Menge von 0,5 ml pro Dosis abgefüllt. Es muss darauf geachtet werden, dass die Vakzine während der Abfüllung geschüttelt wird.

Die Gesamtzahl der inaktivierten Mikroorganismen in einer Dosis - d.h. 0,5 ml - beträgt ca. $1 \times 10^9$.

Geprüft wird die Vakzine schliesslich
- auf Sterilität gemäss der Europäischen Pharmakopöe (2. Ausgabe, 1. Nachtrag 1980),
- auf Toxizität gemäss der Vorschrift der Weltgesundheitsorganisation (Gewichtszunahme bei der Maus),
- auf abnormale Toxizität gemäss der Europäischen Pharmakopöe.

Die Vakzine kann auch, an Stelle der zuvor beschriebenen flüssigen Form, in lyophilisierter Form dargeboten werden. Die Herstellung erfolgt wie im folgenden beschrieben.

Die konzentrierte, inaktivierte Suspension, wie beim obigen Herstellungsverfahren erhalten, wird so verdünnt, dass sie in 0,5 ml ca. $1 \times 10^9$ inaktivierte Bakterien der bereits angegebenen Zusammensetzung enthält. Zur Verdünnung wird eine 1%ige Lösung von Humanalbumin oder vom Plasmaersatz Haemaccel® (Hersteller: Behringwerke AG, 3500 Marburg, BRD) mit 0,01 % Thiomersal in 0,85 n-NaCl-Lösung verwendet.

2 ml Fläschchen werden unter sterilen Bedingungen mit 0,5 ml der oben beschriebenen verdünnten Suspension beschickt, bei ca. -45 °C gefroren, danach unter Vakuum in einem Lyophilisationsapparat getrocknet. Die Trocknungstemperatur sollte +36 °C nicht überschreiten. Der ganze Lyophilisierungsprozess dauert 24 Stunden. Nach der Lyophilisierung werden die Fläschchen unter Stickstoffatmosphäre mit Gummistopfen verschlossen und mit Aluminiumkappen versehen.

Zur Einführung des Aluminiumphosphates und gleichzeitig als Lösungsmittel für die Wiederauflösung vor der Verabreichung wird ein wässriges Aluminiumphosphat-Gel mit einer Konzentration an $AlPO_4$ von 2

mg/ml verwendet.

Die Zubereitung des Aluminiumphosphat-Gels ist im vorhergehenden Herstellungsverfahren beschrieben worden.

Das so gewonnene Aluminiumphosphat-Gel wird, bevor es in die Ampullen abgefüllt wird, mit Kochsalzlösung so verdünnt (ca. 12 mal), dass eine Endkonzentration von 2 mg/ml vorhanden ist.

Die Abfüllmenge in Ampullen von 1 ml Kapazität beträgt 0,5 ml.

Zur Prüfung der Vakzine auf Haltbarkeit wurde ihre Fähigkeit zur Antikörperbildung bei der Maus zweimal bestimmt, und zwar unmittelbar nach der Herstellung, am 3. Dezember 1981, und zwei Jahre später, am 7. Dezember 1983.

Immunisierung der Mäuse :

Sechs Gruppen zu je 10 Mäusen (männlich, NMRI-Stamm, Gewicht 16-20 g) wurden intraperitoneal mit der Vakzine immunisiert. Zwei Dosen (0,5 ml der Vakzine) wurden in einem Abstand von zwei Wochen injiziert. Den als Kontrollgruppe dienenden 20 Mäusen aus derselben Zucht und vom selben Gewicht wurde nur Aluminiumphosphatgel (0,5 ml) injiziert. Zwei Wochen nach der zweiten Injektion wurde Blut aseptisch entnommen und nach Gruppen gepoolt. Nach der Koagulation wurde das Serum von jedem Pool durch Zentrifugieren gewonnen und bei -22 °C tiefgefroren aufbewahrt bis zur serologischen Prüfung.

Vorbereitung der Antigene (Agglutinogene) :

Neun homologe Bakterienstämme, die in der Vakzine enthalten sind, wurden für die Antigenherstellung verwendet; der Stamm Ec 654 war nicht agglutinierbar (R Form) und konnte nicht als Agglutinogen verwendet werden. Die lyophilisierten Kulturen von Bakterienstämmen wurden mit 9 ml Nährboden (veal infusion broth - Difco) suspendiert und die Kultur bei 37 °C während 24 Stunden bebrütet. Die zweite Subkultur wurde für die Antigenvorbereitung verwendet. 600 ml Nährboden (veal infusion broth) wurden mit Saatkultur inokuliert (jeder Stamm separat). Die Kulturen wurden bei 37 °C während 36 Stunden bebrütet. Danach wurde Formalin bis zu einer Endkonzentration von 0,1 % zugegeben. Die Kulturen wurden bei 37 °C während 7 Tagen inkubiert. Jede Kultur wurde danach auf Abwesenheit lebender Bakterien und auf Sterilität geprüft. Die inaktivierte Bakteriensuspension wurde in einer Kühlzentrifuge (Sorval) eine Stunde lang bei 3000 UpM zentrifugiert. Das Sediment wurde in Phosphatpuffer-Kochsalzlösung (pH 7,2) resuspendiert, so dass eine Konzentration von ca. $20 \times 10^9$ Bakterien in einem Milliliter erreicht wurde. Für die Agglutinationsprobe wurden ca. $2 \times 10^9$ Bakterien/ml als Agglutinogen verwendet.

Agglutinations-Test :

Die Mäuseseren wurden in 1:2 Verdünnungsschritten mit phosphatgepufferter physiologischer Kochsalzlösung vom pH 7,2 verdünnt. In Kahn-Röhrchen wurden je 0,3 ml Serumverdünnung mit demselben Volumen Antigen gemischt. Ein Röhrchen ohne Serumzugabe diente als Antigen-Kontrolle. Nach 18 Stunden bei 37 °C und weiteren 24 Stunden bei 4 °C wurde die Reaktion abgelesen. Als Titer des jeweiligen Serums wurde die höchste Verdünnung angenommen, in der noch sichtbare Agglutination der Bakterien festgestellt werden konnte. In allen Ansätzen wurden negative und positive Seren als Kontrollen mitgeführt.

Für die Agglutinintiter der Mäuseseren wurden folgende geometrische Mittelwerte gefunden:

| Antigen | Titer (reziproke Werte) | |
|---|---|---|
| | geprüft 1981 | geprüft 1983 |
| E. coli 455 UB | 1015,5 | 905,1 |
| E. coli 525 UB | 905,1 | 1015,5 |
| E. coli 560 UB | 1015,5 | 905,1 |
| E. coli 616 UB | 905,1 | 806,3 |
| E. coli 719 UB | 1280 | 1280 |
| Klebsiella pneumoniae 16B | 201,6 | 201,6 |
| Proteus mirabilis 63B | 160,0 | 142,5 |
| Proteus morganii 58B | 113,1 | 113,1 |
| Streptococcus faecalis 676 | 71,3 | 71,3 |

Aus diesen Ergebnissen lässt sich ersehen, dass die Vakzine bei einer Lagerung bei 4 °C ihre volle Wirksamkeit mindestens zwei Jahre lang behält.

Die Indikationen der Vakzine sind insbesondere die Cystitis, die Prostatitis, die Cysto-pyelitis und die Pyelonephritis.

Patienten, die keinen akuten Fieberzustand aufweisen (Kontraindikation), erhalten in Abständen von je 1 bis 2 Wochen insgesamt 3 intramuskuläre Injektionen mit je 0,5 ml Impfstoff. Bei Auftreten von starken Impfreaktionen soll die Behandlung abgebrochen werden.

Ein Jahr danach soll eine Rappelinjektion zur Auffrischung, ebenfalls in einer Dosis von 0,5 ml, verabreicht werden.

Mit der Vakzine sind bisher in verschiedenen Spitälern 383 Patienten mit symptomatischen, bakteriologisch gesicherten Harnwegsinfektionen geimpft und danach während über einem Jahr weiter beobachtet worden. Die Ergebnisse zeigen, dass die Impfung eine mindestens ein Jahr lang anhaltende Verbesserung des Widerstandes der Harnwege bewirkt, welche einen weitgehenden Schutz vor rezidivierenden Harnwegsinfektionen zur Folge hat. Die Ergebnisse der klinischen Untersuchungen werden in der folgenden Tabelle zusammengefasst.

## Anzahl der Rezidive/Reinfekte während der Beobachtungszeit von 1 bis 12 Monaten

| Untersucher | O<br>Studienbeginn | I<br>1 bis 2<br>Monate | VI<br>7 bis 12<br>Monate |
|---|---|---|---|
| Li/BS | 62/62 | 6/62 | —— |
|  | 100 % | 9,3 % | —— |
| Li/SH | 118/118 | 12/113 | 2/115 |
|  | 100 % | 10,6 % | 1,7 % |
| Rü/H, DE | 203/203 | 15/183 | 3/194 |
|  | 100 % | 8,2 % | 1,5 % |
| Total Vakzinierte | 383/383 | 33/358 | 5/309 |
|  | 100 % | 9,2 % | 1,6 % |
| Kontrolle | 198/198 | 26/148 | 18/146 |
|  | 100 % | 17,6 % | 12,3 % |

Die Verträglichkeit ist als gut zu bezeichnen. Ernsthafte Nebenwirkungen traten keine auf. Die Vakzine ist auch in der Schwangerschaft problemlos anwendbar; die Erfahrungen an über 100 Schwangeren belegen dies.

Zum Schluss sollen wesentliche Merkmale und Vorteile der Erfindung sowie auch die Ergebnisse weiterer, hier nicht näher beschriebenen pharmakologischen und klinischen Untersuchungen mit der neuen Vakzine zusammengefasst werden :

Die quantitative Zusammensetzung der 10 verschiedenen Stämme ist nach der Häufigkeitsverteilung der Erreger der Harnwegsinfektionen zusammengestellt : 75 % der Stämme sind E.coli-Stämme (gleichmässig vertreten), 25 % Proteus mirabilis-, Proteus morganii-, Klebsiella pneumoniae- und Streptococcus faecalis-Stämme.

Diese uropathogenen Stämme sind so inaktiviert, dass sie die schutzbewirkenden Antigene enthalten, wie z.B. fimbriale Antigene und O-Antigene. Die ausgewählten Stämme als Antigene zeigen, wenn sie in einem Mäuseschutztest geprüft werden, auch einen gekreuzten Schutz gegen die heterologen Stämme derselben Erregerart.

Wie mit dem Mausgewichtzunahmetest (mouse weight gain test) festgestellt wurde, sind die inaktivierten Antigene, die in der Vakzine vertreten sind, nach einer Lagerung von wenigstens 6 Monaten in einem Kühlraum, deutlich weniger toxisch geworden.

Die Vakzine ruft die Immunantwort in geimpften Tieren (Mäusen, Kaninchen) wie auch bei geimpften Menschen hervor, schützt Mäuse vor einer letalen Infektion, die Ratten vor einer experimentellen Pyelonephritis.

Die quantitative und qualitative Zusammensetzung entspricht der Antigenmenge, welche noch eine gute Immunantwort hervorruft und bei Patienten keine schweren oder keine Nebenwirkungen verursacht.

Die Vakzine ruft auch sekretorisches Immunoglobulin A im Urin von geimpften Patienten hervor.

Die Vakzine heilt an einer Harnwegsinfektion erkrankte Personen; rezividierende Patienten schützt sie mindestens 12 Monate lang vor einer Reinfektion.

Die Vakzine kann in flüssiger oder lyophilisierter (gefriergetrockneter) Form hergestellt werden. Bei der lyophilisierten Form wird ein Aluminiumphosphatgel als Lösungsmittel zur Wiederauflösung vor der Verabreichung verwendet.

**Patentansprüche**

1. Vakzine zur kurativen und prophylaktischen Behandlung der Harnwegsinfektionen beim Menschen, welche besteht aus (1) inaktivierten Bakterien, die aus Kulturen von 8 bis 14 aus dem Harn von an einer Infektion der Harnwege leidenden Menschen isolierten uropathogenen Bakterienstämmen der Arten :
   - Escherichia coli
   - Klebsiella pneumoniae
   - Proteus mirabilis
   - Proteus morganii und
   - Streptococcus faecalis

   stammen und in einer Menge von etwa 50 Millionen bis 500 Millionen Keime je Stamm per ml vorliegen, wobei die Hälfte bis Dreiviertel der verwendeten Stämme der Art Escherichia coli angehören, und (2) Aluminiumphosphat in kolloidaler Form in einer Menge von 1,5 bis 10 mg AlPO₄ per ml, in einer sterilen isotonischen Lösung suspendiert.

2. Vakzine nach Anspruch 1, in welcher die inaktivierten Bakterien aus 6 Stämmen der Art Escherichia coli und je 1 Stamm der vier anderen oben genannten Arten stammen.

3. Vakzine nach Anspruch 1, in welcher die inaktivierten Bakterien aus folgenden Stämmen stammen (Firmainterne Bezeichnung bzw. DSM-Eingangsnummer):
   - Escherichia coli Ec 455 UB, 525 UB, 560 UB, 616 UB, 654 UB, 719 UB (DSM 3229 bis DSM 3234)
   - Klebsiella pneumoniae 16 B (DSM 3235).
   - Proteus mirabilis 63 B (DSM 3238)`
   - Proteus morganii 58 B (DSM 3237).
   - Streptococcus faecalis 676 (DSM 3236).

4. Vakzine nach einem der Ansprüche 1 bis 3, in welcher die inaktivierten Bakterien zusammen in einer Menge von etwa 2 x 10⁹ Keime per ml vorliegen.

5. Vakzine nach einem der Ansprüche 1 bis 4, in welcher ausserdem ein Konservierungsmittel vorliegt.

6. Verfahren zur Herstellung der Vakzine nach Anspruch 1, dadurch gekennzeichnet, dass man 8 bis 14 uropathogene Bakterienstämme der Arten:
   - Escherichia coli
   - Klebsiella pneumoniae
   - Proteus mirabilis
   - Proteus morganii und
   - Streptococcus faecalis,

   welche aus dem Harn von an einer Infektion der Harnwege leidenden Menschen isoliert worden sind und von welchen die Hälfte bis Dreiviertel der Art Escherichia coli angehören, jeweils für sich allein auf einem geeigneten Nährboden züchtet, nach Abschluss der Züchtung das jeweils gebildete biologische Material abtrennt und nach bekannten Methoden inaktiviert, die aus den einzelnen Stämmen erhaltenen und inaktivierten Bakterien miteinander in solchen Mengen vermischt und mit einer sterilen isotonischen Lösung soweit verdünnt, dass etwa 50 Millionen bis 500 Millionen Keime je Stamm per ml vorliegen, und mit Aluminiumphosphat in kolloidaler Form bis zu einer Konzentration von 1,5 bis 10 mg AlPO₄ per ml versetzt.

7. Verfahren nach Anspruch 6, dadurch gekennzeichnet, dass man für die Züchtung 6 Stämme der Art Escherichia coli und je 1 Stamm der vier anderen oben genannten Arten einsetzt.

8. Verfahren nach Anspruch 6, dadurch gekennzeichnet, dass man für die Züchtung folgende Stämme einsetzt (firmainterne Bezeichnung bzw. DSM-Eingangsnummer):
   - Escherichia coli Ec 455 UB, 525 UB, 560 UB, 616 UB, 654 UB, 719 UB (DSM 3229 bis DSM 3234)
- Klebsiella pneumoniae 16 B (DSM 3235)
- Proteus mirabilis 63 B (DSM 3238)
- Proteus morganii 58 B (DSM 3237)
- Streptococcus faecalis 676(DSM 3236)`

9. Verfahren nach einem der Ansprüche 6 bis 8, dadurch gekennzeichnet, dass man das biologische Material durch Erhitzen einer wässrigen Suspension auf 55 bis 60 °C während etwa 1 Stunde, durch Behandlung mit Formaldehyd oder durch Bestrahlung mit γ-Strahlen inaktiviert.

**Claims**

1. A vaccine for curative and prophylactic treatment of urinary tract infections in humans, which consists of (1) inactivated bacteria which originate from cultures of 8 to 14 uropathogenic bacteria strains isolated from the urine of persons suffering from an infection of the urinary tract, of the species:
   - Escherichia coli,
   - Klebsiella pneumoniae,
   - Proteus mirabilis,
   - Proteus morganii and
   - Streptococcus faecalis,

   and are present in an amount of about 50 million to 500 million germs of each strain per ml, one half to three quarters of the strains used belonging to the Escherichia coli species, and (2) aluminum phosphate in colloidal form in an amount of 1.5 to 10 mg of $AlPO_4$ per ml, suspended in a sterile isotonic solution.

2. A vaccine as claimed in claim 1, in which the inactivated bacteria originate from 6 strains of the species Escherichia coli and 1 strain each of the other four abovementioned species.

3. A vaccine as claimed in claim 1, in which the inactivated bacteria originate from the following strains (in-house designation and DSM receipt number, respectively):
   - Escherichia coli Ec 455 UB, 525 UB, 560 UB, 616 UB, 654 UB, 719 UB (DSM 3229 to DSM 3234),
   - Klebsiella pneumoniae 16 B (DSM 3235),
   - Proteus mirabilis 63 B (DSM 3238),
   - Proteus morganii 58 B (DSM 3237),
   - Streptococcus faecalis 676 (DSM 3236).

4. A vaccine as claimed in any one of claims 1 to 3, in which the inactivated bacteria are present in a total amount of about $2 \times 10^9$ germs per ml.

5. A vaccine as claimed in any one of claims 1 to 4, in which a preservative is also present.

6. A process for the preparation of a vaccine as claimed in claim 1, which comprises culturing, by themselves on a suitable nutrient medium, each of the 8 to 14 uropathogenic bacteria strains of the species
   - Escherichia coli,
   - Klebsiella penumoniae,
   - Proteus mirabilis,
   - Proteus morganii and
   - Streptococcus faecalis,

   which have been isolated from the urine of persons suffering from an infection of the urinary tract and of which half to three quarters belong to the species Escherichia coli, and, when culturing is concluded, removing the particular biological material formed and inactivating it by known methods, mixing amounts of the inactivated bacteria obtained from the individual strains with one another and diluting the mixture with an amount of a sterile isotonic solution such that about 50 million to 500 million germs of each strain are present per ml, and adding aluminum phosphate in colloidal form up to a concentration of 1.5 to 10 mg of $AlPO_4$ per ml.

7. The process as claimed in claim 6, wherein 6 strains of the species Escherichia coli and 1 strain of each of the other four abovementioned species are used for culture.

8. The process as claimed in claim 6, wherein the following strains are used for culture (in-house designation and DSM receipt number, respectively):
   - Escherichia coli Ec 455 UB, 525 UB, 560 UB, 616 UB, 654 UB, 719 UB (DSM 3229 to DSM 3234),
   - Klebsiella pneumoniae 16 B (DSM 3235),
   - Proteus mirabilis 63 B (DSM 3238),
   - Proteus morganii 58 B (DSM 3237),
   - Streptococcus faecalis 676 (DSM 3236).

9. The process as claimed in any one of claims 6 to 8, wherein the biological material is inactivated by heating an aqueous suspension to 55 to 60°C for about 1 hour, by treatment with formaldehyde or by irradiation with γ-rays.

## Revendications

1. Vaccin pour le traitement curatif et prophylactique des infections des voies urinaires chez l'homme, qui consiste en (1) des bactéries inactivées qui proviennent de cultures des souches uropathogènes suivantes, isolées à partir de l'urine d'êtres humains atteints d'une infection des voies urinaires:
   - Escherichia coli
   - Klebsiella pneumoniae
   - Proteus mirabilis
   - Proteus morganii
   - Streptococcus faecalis
   et qui sont présentes en une quantité d'environ 50 millions à 500 millions de germes par ml, étant entendu que de la moitié jusqu'aux trois-quarts des souches utilisées appartiennent à l'espèce Escherichia coli, et (2) du phosphate d'aluminium sous forme colloïdale en une quantité de 1,5 à 10 mg de AlPO₄ par ml, en suspension dans une solution isotonique stérile.

2. Vaccin selon la revendication 1, dans lequel les bactéries inactivées proviennent de 6 souches de l'espèce Escherichia coli et d'une souche chacune des quatre autres espèces mentionnées ci-dessus.

3. Vaccin selon la revendication 1, dans lequel les bactéries inactivées proviennent des souches suivantes (désignation interne de la société et numéro d'accès DSM, respectivement):
   - Escherichia coli Ec 455 UB, 525 UB, 560 UB, 616 UB, 654 UB, 719 UB (DSM 3229 jusqu'à DSM 3234)
   - Klebsiella pneumoniae 16 B (DSM 3235)
   - Proteus mirabilis 63 B (DSM 3238)
   - Proteus morganii 58 B (DSM 3237)
   - Streptococcus faecalis 676 (DSM 3236).

4. Vaccin selon l'une des revendications 1 à 3, dans lequel les bactéries inactivées sont présentes, prises ensemble, en une quantité d'environ $2 \times 10^9$ germes par ml.

5. Vaccin selon l'une des revendications 1 à 4, dans lequel, en outre, un agent de conservation est présent.

6. Procédé de préparation du vaccin selon la revendication 1, caractérisé en ce que l'on cultive sur un milieu nutritif approprié, chaque souche pour elle-même, de 8 à 14 souches de bactéries uropathogènes des espèces:
   - Escherichia coli
   - Klebsiella pneumoniae
   - Proteus mirabilis
   - Proteus morganii et
   - Streptococcus faecalis,
   qui ont été isolées à partir de l'urine d'êtres humains atteints d'une infection des voies urinaires et dont

19

de la moitié jusqu'aux trois-quarts appartiennent à l'espèce Escherichia coli, on sépare le matériel biologique produit à chaque fois après achèvement de la culture et on l'inactive selon des méthodes connues, on mélange les unes avec les autres les bactéries inactivées obtenues à partir des souches individuelles, en des quantités telles et on les dilue avec une solution isotonique stérile de façon telle qu'il y ait de 50 millions à 500 millions de germes par ml pour chaque souche, et on traite par du phosphate d'aluminium sous forme colloïdale jusqu'à une concentration de 1,5 à 10 mg de ALPO$_4$ par ml.

7. Procédé selon la revendication 6, caractérisé en ce que l'on met en jeu pour la culture 6 souches de l'espèce Escherichia coli et une souche chacune des quatre autres espèces mentionnées ci-dessus.

8. Procédé selon la revendication 6, caractérisé en ce que l'on met en jeu pour la culture les souches suivantes (désignation interne de la société et numéro d'accès DSM, respectivement):
   - Escherichia coli Ec 455 UB, 525 UB, 560 UB, 616 UB, 654 UB 719 UB (DSM 3229 jusqu'à DSM 3234)
   - Klebsiella pneumoniae 16 B (DSM 3235)
   - Proteus mirabilis 63 B (DSM 3238)
   - Proteus morganii 58 B (DSM 3237)
   - Streptococcus faecalis 676 (DSM 3236).

9. Procédé selon l'une des revendications 6 à 8, caractérisé en ce que l'on inactive le matériel biologique par chauffage d'une suspension aqueuse à 55-60°C pendant environ une heure, par traitement au formaldehyde ou par irradiation avec des rayons $\gamma$.